(19) Europäisches Patentamt / European Patent Office / Office européen des brevets

(11) **EP 3 532 137 B1**

(12) **EUROPEAN PATENT SPECIFICATION**

(45) Date of publication and mention
of the grant of the patent:
**25.05.2022 Bulletin 2022/21**

(21) Application number: **16794447.9**

(22) Date of filing: **27.10.2016**

(51) International Patent Classification (IPC):
**A61M 16/00** *(2006.01)*

(52) Cooperative Patent Classification (CPC):
**A61M 16/00;** A61M 2016/0015

(86) International application number:
**PCT/SE2016/051050**

(87) International publication number:
**WO 2018/080358 (03.05.2018 Gazette 2018/18)**

(54) **BIOELECTRICALLY CONTROLLED VENTILATION**

BIOELEKTRISCH GESTEUERTER VENTILATION

VENTILATION À COMMANDE BIOÉLECTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.09.2019 Bulletin 2019/36**

(73) Proprietor: **Maquet Critical Care AB
171 06 Solna (SE)**

(72) Inventor: **LARSSON, Åke
175 64 Järfälla (SE)**

(74) Representative: **Zacco Sweden AB
P.O. Box 5581
114 85 Stockholm (SE)**

(56) References cited:
**WO-A1-2014/128668      US-A- 5 390 666
US-A1- 2014 190 485**

**Description**

TECHNICAL FIELD

The present disclosure relates to a breathing apparatus, a method, and a computer program for providing bioelectrically controlled ventilation to a patient.

BACKGROUND

In the field of mechanical ventilation, there are various techniques for adjusting the ventilation pattern provided by the ventilator to the patient's own breathing efforts. Ventilation modes in which the ventilator adapts the supply of breathing gas to detectable breathing efforts of a ventilated patient are generally referred to as modes of assisted or supported ventilation. A ventilator that is operated in such a ventilation mode is said to provide support ventilation to the patient.

In recent years there has evolved techniques for neurally adjusted ventilation, i.e., techniques in which the ventilation pattern provided to the patient by the ventilator is adapted to the breathing efforts of the patient by controlling the supply of breathing gas by the ventilator based on bioelectrical signals indicating at least the points in time at which there is a desire of the patient to inhale and/or exhale. An example of such a technique is the now clinically well-established technique of neurally adjusted ventilatory assist (NAVA).

The act of taking a breath is controlled by the respiratory centre of the brain, which decides the characteristics of each breath, including timing and size. The respiratory centre sends a signal along the phrenic nerve, excites the diaphragm muscle cells, leading to muscle contraction and descent of the diaphragm dome. As a result, the pressure in the airway drops, causing an inflow of air into the lungs.

With NAVA, the electrical activity of the diaphragm (Edi) is captured, fed to a NAVA-enabled ventilator and used to assist the patient's breathing in synchrony with and in proportion to the patient's own breathing efforts. As the work of the ventilator and the diaphragm is controlled by the same signal, coupling between the diaphragm and the NAVA-enabled ventilator is synchronized simultaneously.

The NAVA technology is further described in e.g. WO 1998/48877, WO 1999/62580, WO 2006/131149, and WO 2008/131798.

In some situations it may be difficult to detect or reliably detect the bioelectric signal used to control the ventilation in bioelectrically controlled ventilation modes, such as the NAVA mode. This may be due to problems in capturing the signal, but it may also be due to a disability of the patient with respect to generating the bioelectric signal. In such situations, in order to ensure sufficient ventilation of the patient, the breathing apparatus may be configured to switch from the bioelectrically controlled ventilation mode to a backup ventilation mode that is independent of the bioelectric signal.

US 8,469,026 describes a breathing apparatus that is configured to switch from a bioelectrically controlled support mode to a backup mode that is independent of the bioelectric signal if the bioelectric signal is not present at a control input of the breathing apparatus. The breathing apparatus then remains in the backup mode until the bioelectric signal is regained, whereupon the breathing apparatus returns to the bioelectrically controlled ventilation mode.

A challenge with the above described ventilation scheme is the discomfort sometimes experienced by the patient due to the sometimes abrupt transition between the bioelectrically controlled ventilation mode and the backup mode. Another challenge is to ensure that the ventilation provided by the breathing apparatus is optimally adapted to the natural breathing cycles and the actual breathing needs of the patient. Yet another challenge is to ensure that the ventilation is optimally adapted to the pulmonary mechanics of the patient.

SUMMARY

The invention is defined by the appended claims.

According to one aspect of the present disclosure there is provided a breathing apparatus, such as a ventilator or an anaesthesia machine, configured to ventilate a patient in a mode in which a patient-triggered breath is delivered to the patient upon detection of a bioelectrical trigger event indicative of a spontaneous breathing effort by the patient. The breathing apparatus is further configured to monitor a level of ventilation of the patient, e.g., the minute ventilation of the patient, and to deliver a non-patient triggered breath to the patient when the level of ventilation falls below a minimum ventilation threshold value, representing a mandatory minimum level of ventilation of the patient.

This means that the breathing apparatus typically delivers patient-triggered breaths that are triggered by bioelectric activity indicative of spontaneous breathing efforts by the patient as long as the level of ventilation of the patient remains above a mandatory minimum level of ventilation. Should the monitored level of ventilation fall below the mandatory minimum level, a non-patient triggered breath is delivered to the patient to keep the level of ventilation above the mandatory minimum level of ventilation. Consequently, contrary to known modes of bioelectrically controlled ventilation, the proposed bioelectrically controlled mode of ventilation comes with a minimum ventilation guarantee.

The triggering of a non-patient triggered breath is typically completely independent of the bioelectrical signal, meaning that the breath is not delivered upon detection of an event that is indicative of spontaneous breathing activity of the patient.

The non-patient triggered breath may be a controlled breath defined by preset parameters, or it may be a support breath defined by parameters that are, at least to some extent, adjustable and dependent on the pulmonary mechanics of the patient. The non-patient triggered breath is a ventilator-initiated breath, which hereinafter is referred to as a "forced breath" as it is forced onto the patient when the monitored level of ventilation falls below the minimum ventilation threshold value. This is in contrast to the patient-triggered breath, which may be referred to as an "unforced breath" since it is triggered by the patient's own breathing efforts.

It should be noted that the delivery of a forced breath does not imply that the ventilation mode of the breathing apparatus is changed from one mode of ventilation to another. Instead, delivery of any forced breaths should be regarded as intermittent delivery of forced breaths during a bioelectrically controlled ventilation mode to assure that the ventilation of the patient is maintained above a minimum level of ventilation. Furthermore, it should be noted that the forced breaths are not mandatory breaths in the meaning of being delivered at fixed points in time, or with a preset time delay in relation to a preceding breath. Instead, the timing of delivery of a forced breath is adjustable and dependent on the current level of ventilation of the patient.

In the proposed type of bioelectrically controlled ventilation mode, at least the time of delivery of unforced breaths is controlled based on the captured bioelectric signal. Preferably, also the magnitude (in terms of tidal volume, airway flow or airway pressure) of the unforced breaths is controlled based on the bioelectric signal. For example, as long as the bioelectrical signal is available, the bioelectrically controlled ventilation mode may cause the breathing apparatus to operate in accordance with a breathing apparatus operating in the well-known ventilation mode of neurally adjusted ventilatory assist (NAVA), in which breaths are delivered to the patient in synchrony with and in proportion to a bioelectric signal indicative of the patient's breathing effort.

The bioelectric signal may be any type of measurable bioelectrical signal indicative of the breathing efforts of the patient. Non-exclusive examples of usable bioelectric signals are EMG (electromyogram) signals representing the electrical activity of the diaphragm or muscles in the upper airways (e.g., the laryngopharyngeal region), and EEG (electroencephalography) signals representing the electrical activity of respiratory centres of the brain.

In an exemplary and non-limiting embodiment, the forced breath that is delivered to the patient when the monitored level of ventilation falls below the minimum ventilation threshold values is a type of pressure-support breath. In pressure support ventilation, the delivery of a breath is typically triggered by detection of a pneumatic trigger event, such as a pressure drop in the breathing circuit, whereby a preset pressure is applied to the airways of the patient for a time period determined by a so called cycle-off criterion. The cycle-off criterion is typically a threshold value for inspiratory flow and is normally set to a certain percentage of the maximum inspiratory flow, e.g., a value within the range of 30-70% of maximum inspiratory flow. The forced breath may be a type of pressure-support breath that, contrary to conventional pressure-support breaths, is not triggered by a pneumatic trigger event. Instead, the forced type of pressure-support breath is triggered by the trigger condition for the monitored level of ventilation of the patient, whereas the duration of the forced breath (e.g. the duration of the inspiratory phase of the breath) may be determined by a conventional cycle-off criterion for inspiratory flow. This means that although being a forced breath in the meaning of being non-patient triggered, the duration of the forced breath may be adapted to the pulmonary mechanics of the patient. In this way, the discomfort and the pulmonary stress caused by the intermittent delivery of forced breaths can be minimized, thereby minimizing the risk of adversely affecting the spontaneous breathing of the patient and the time of weaning from mechanical ventilation.

In other exemplary embodiments, the forced breath may be any of a pressure controlled, volume controlled or Bilevel Positive Airway Pressure (BiPap) controlled breath.

In some embodiments, the breathing apparatus may be configured to use a second and time-based trigger condition for delivery of forced breaths, in parallel with the first trigger condition for the monitored level of ventilation of the patient. This means that the breathing apparatus may be configured to deliver a forced breath to the patient when any of the first and the second trigger conditions is met. The time-based trigger condition may be a maximum time for the time elapsed since delivery of a preceding breath, thereby defining a maximum time interval between consecutive breaths. This has the effect of ensuring that the patient receives a breath within a reasonable period of time, even if the overall ventilation of the patient is well above the minimum ventilation threshold value. In an exemplary embodiment, the maximum time may be set to 15 seconds.

In some embodiments, the breathing apparatus may be configured to also deliver breaths to the patient upon detection of a pneumatic trigger event indicative of a spontaneous breathing effort by the patient. This has the effect of providing the breathing apparatus with a backup function in the proposed mode of bioelectrically controlled ventilation, which backup function allows breaths to be delivered in synchrony with the patient's spontaneous breathing efforts also in situations where the bioelectrical signal cannot be detected, or cannot be reliably detected.

In some embodiments, the breathing apparatus may be configured to detect both bioelectrical and pneumatic trigger events, and to deliver breaths to the patient on a first-come first-serve basis, meaning that a breath is delivered to the patient as soon as any of a bioelectrical or a pneumatic trigger event is detected. Since both bioelectrical and pneumatic trigger events will cause breaths to be delivered to the patient and, thus, prevent the monitored level of ventilation from falling below the minimum ventilation threshold value, this has the effect of minimizing undesired delivery of forced breaths.

In other embodiments, the breathing apparatus may be configured to deliver breaths only in response to detection of bioelectrical trigger events (and not pneumatic trigger events) for a first period of time, and to deliver breaths in response to detection of any of a bioelectrical or a pneumatic trigger events during a second period of time following the first period of time. This has the additional effect of giving preference to bioelectrical triggering of breaths, which allows the characteristics of the breaths to be adapted to the bioelectrical signal and, thus, to what is considered to best represent the true breathing needs of the patient.

The pneumatically triggered breaths may be any type of breaths, such as traditional pressure support or volume support breaths.

The monitored level of ventilation is preferably a measure of the volume of gas inhaled and/or exhaled by the patient per time unit.

In some embodiments, the monitored level of ventilation of the patient is the minute ventilation of the patient. The breathing apparatus may thus be configured to monitor the minute ventilation of the patient, and to deliver a forced breath to the patient when the minute ventilation falls below a certain threshold value, so as to maintain a mandatory minute ventilation (MMV). The monitored minute ventilation may be a running minute ventilation of the patient, meaning that it is a measure of the minute ventilation of the patient over a running time window. The duration of the running time window may, for example, be a preset time period or a time period defined by a predetermined number of breaths.

According to another aspect of the present disclosure there is provided a method of providing bioelectrically controlled ventilation to a patient by ventilating the patient in a ventilation mode in which a patient-triggered breath (i.e., an unforced breath) is delivered to the patient upon detection of a bioelectrical trigger event indicative of a spontaneous breathing effort by the patient. The method further comprises the steps of monitoring a level of ventilation of the patient, e.g., the minute ventilation of the patient, and delivering a non-patient triggered breath (i.e., a forced breath) to the patient when the level of ventilation falls below a certain threshold value.

In some embodiments, the method may comprise the step of delivering a forced breath also when a certain period of time has elapsed since a preceding breath was delivered to the patient.

In some embodiments, the method may comprise the step of delivering breaths to the patient also upon detection of a pneumatic trigger event indicative of a spontaneous breathing effort by the patient.

In some embodiments, the forced breath may be a type of pressure-support breath, whereby the method may comprise the step of adapting the duration of the forced type of pressure-support breath to the pulmonary mechanics of the patient, e.g., by employing a cycle-off criterion for inspiratory flow.

According to another aspect of the present disclosure, there is provided a computer program for causing a breathing apparatus to provide bioelectrically controlled ventilation to a patient by ventilating the patient in a ventilation mode in which a patient-triggered breath (i.e., an unforced breath) is delivered to the patient upon detection of a bioelectrical trigger event indicative of a spontaneous breathing effort by the patient. The computer program comprises computer-readable code segments which, when executed by a processor of the breathing apparatus, cause the breathing apparatus to monitor a level of ventilation of the patient, e.g., the minute ventilation of the patient, and deliver a non-patient triggered breath (i.e., a forced breath) to the patient when the level of ventilation falls below a certain threshold value.

The computer program may further comprise code segments which, when executed by the processor, cause the breathing apparatus to carry out any of the method steps described above.

According to yet another aspect of the present disclosure, there is provided a computer program product comprising a non-volatile memory that stores the above mentioned computer program.

Further aspects and advantages of the novel type of bioelectrically controlled ventilation mode will become apparent in view of the detail description following hereinafter.

BRIEF DESCRIPTION OF THE DRAWINGS

Embodiments of this disclosure will become more fully understood from the detailed description provided hereinafter and the accompanying drawings which are given by way of illustration only. In the different drawings, same reference numerals correspond to the same element.

Fig. 1 illustrates a breathing apparatus according to an exemplary embodiment of the present disclosure.

Fig. 2 illustrates the minute ventilation of a patient as a function of time in an exemplary scenario in which the patient is ventilated using the proposed bioelectrical mode of ventilation.

Fig. 3 illustrates the minute ventilation of a patient as a function of time in another exemplary scenario in which the patient is ventilated using the proposed bioelectrical mode of ventilation.

Fig. 4 illustrates pressure-time and flow-time relationships of a forced breath according to an exemplary embodiment of the present disclosure.

Fig. 5 is a flow chart illustrating a method of providing bioelectrically controlled ventilation to a patient according to an exemplary embodiment of the present disclosure.

Fig. 6 is a flow chart illustrating a method of providing bioelectrically controlled ventilation to a patient according to another exemplary embodiment of the present disclosure.

DETAILED DESCRIPTION

A novel type of bioelectrically controlled ventilation mode with a minimum minute ventilation guarantee will now be described with reference to an exemplary and non-limiting embodiment.

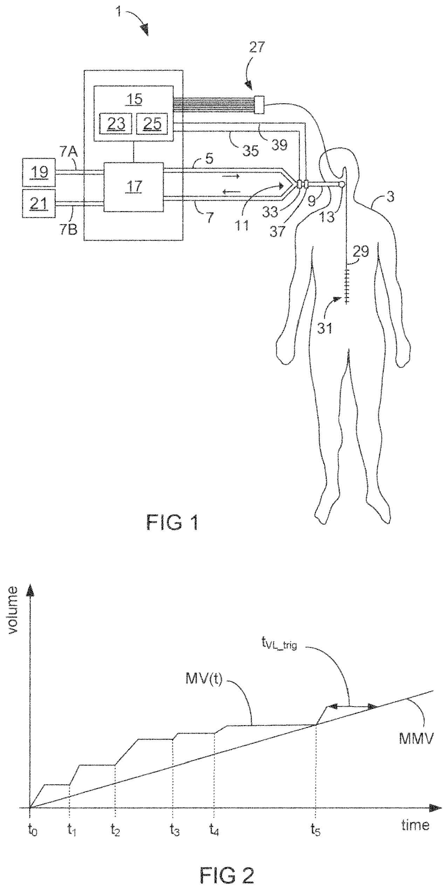

Fig. 1 illustrates a breathing apparatus 1, such as a ventilator or an anaesthesia machine, for providing ventilatory treatment to a patient 3. The breathing apparatus 1 is connected to the patient 3 via an inspiratory line 5 for supplying breathing gas to the patient 3, and an expiratory line 7 for conveying expiration gas away from the patient 3. The inspiratory line 5 and the expiratory line 7 are connected to a common line 9, via a so called Y-piece 11, which common line is connected to the patient 3 via a patient connector 13, such as a facemask or an endotracheal tube.

The breathing apparatus 1 further comprises a control unit 15 for controlling the ventilation of the patient 3 based on preset parameters and/or measurements obtained by various sensors of the breathing apparatus. The control unit 15 controls the ventilation of the patient 3 by controlling a pneumatic unit 17 of the breathing apparatus 1, which pneumatic unit 17 is connected on one hand to one or more gas sources 19, 21 and on the other hand to the inspiratory line 5 for regulating a flow and/or pressure of breathing gas delivered to the patient 3. To this end, the pneumatic unit 17 may comprise various gas mixing and regulating means well known in the art of ventilation, such as gas mixing chambers, controllable gas mixing valves and one or more controllable inspiration valves.

The control unit 15 comprises a processing unit 23, such as a microprocessor, and a non-volatile memory hardware device 25 storing a computer program for controlling the operation of the breathing apparatus 1 in accordance with the principles described herein. Unless stated otherwise, actions and method steps described hereinafter are performed by, or caused by, the control unit 15 of the breathing apparatus 1 upon execution by the processing unit 23 of different code segments of the computer program stored in the memory 25.

The breathing apparatus 1 further comprises a bioelectric sensor arrangement 27 coupled to the control unit 15 of the breathing apparatus 1 and configured to detect bioelectric signals indicative of the patient's efforts to breathe. When the breathing apparatus 1 is operated in the bioelectrically controlled mode of ventilation, the control unit 15 controls the pneumatic unit and ,thus, the ventilation of the patient 3 based on the bioelectric signals detected by the bioelectric sensor arrangement 27.

That the control unit 15 controls the ventilation of the patient 3 based on the bioelectric signal captured by the bioelectric sensor arrangement 27 means that the bioelectric signal is used by the control unit 15 at least for the triggering of breaths that are to be delivered to the patient 3, i.e., for determining the onset time of inspiration phases. The bioelectric signal may also be used by the control unit 15 to control other breath-related parameters, such as the airway pressure applied during the breath, the time for cycle off of the breath, etc. Preferably, the bioelectric signal is used by the control unit 15 to control both the timing and the magnitude of the breaths delivered to the patient 3.

In the exemplary embodiment illustrated in Fig. 1, the bioelectric sensor arrangement 27 is an EMG detector for recording the diaphragm EMG of the patient 3. To this end, the sensor arrangement 27 comprises an oesophageal catheter 29 for capturing myoelectrical signals (EMG signals) from the diaphragm of the patient 3. The catheter 29 comprises a number of electrodes 31 that produce a number of subsignals. The subsignals are then processed by the control unit 15 to calculate a signal, the Edi signal, representing the electrical activity of the diaphragm and so indicative of the patient's efforts to breathe. The Edi signal is then used by the control unit 15 to control the supply of breathing gas to the patient 3 in synchrony with and in proportion to the patient's own efforts to breathe, as described in greater detail, e.g., in WO 1998/48877 and WO 1999/62580.

Although exemplified in form of an oesophageal catheter arrangement, the bioelectric sensor arrangement 27 could be any bioelectric sensor arrangement known in the art for detection of bioelectric signals indicative of a subject's effort to breath. For example, the bioelectric sensor arrangement could comprise a number of surface electrodes placed on the ribcage, the abdomen or in the vicinity of the phrenic nerve of the patient 3 to sense and filter out diaphragmatic EMG signals to be used in the control of the breathing apparatus 1. According to another example, the bioelectric sensor arrangement could be devised to detect laryngopharyngeal EMG signals of the patient 3, and to use the laryngopharyngeal EMG signals for bioelectrical control of the breathing apparatus according to the principles described in the co-pending international application no. PCT/SE2015/050369. According to yet another example, the bioelectric sensor arrangement 27 could be configured to detect EEG signals representing the electrical activity of respiratory centres of the patient's brain, e.g., by means of scalp EEG electrodes, and to use the EEG signals for bioelectric control of the breathing apparatus 1. The use of EEG signals in bioelectric control of a breathing apparatus is further discussed, e.g., in Grave de Peralta et al., Patient Machine Interface for the Control of Mechanical Ventilation Devices, Brain Sci. 2013, 3, 1554-1568, doi:10.3390/brainsci3041554.

In the bioelectrical mode of ventilation, the control unit 15 is further configured to continuously monitor the level of ventilation of the patient 3. To this end, the control unit 15 is configured to monitor at least one parameter that is indicative of the current level of ventilation of the patient 3. Preferably, the at least one monitored parameter is indicative of a volume per time unit of inhaled and/or exhaled gas. For example, the control unit 15 may be configured to monitor the minute ventilation of the patient 3, and to use the minute ventilation as a measure of the level of ventilation of the patient 3.

The minute ventilation may, for example, be determined by the control unit 15 as the volume of gas inhaled (inhaled minute ventilation) or exhaled (exhaled minute ventilation) of the patient 3 per minute. To this end, the breathing apparatus 1 may comprise a flow sensor 33 for measuring inspiratory and/or expiratory flow, whereby the control unit 15 may be configured to calculate the minute ventilation of the patient 3 from measurements obtained by the flow sensor 33. In the exemplary embodiment illustrated in Fig. 1, the flow sensor 33 is located in or close to the Y-piece 11 and configured to measure any or both of the inspiratory flow and the expiratory flow. The measurement signals obtained by the flow sensor 33 are transmitted to the control unit 15 via a signalling line 35, whereby the control unit 15 uses the measurement signals to calculate the minute ventilation of the patient 3. In other exemplary embodiments, flow sensors having the same function may be arranged within a respective inspiratory and expiratory module of the breathing apparatus 1.

The calculated minute ventilation should reflect the current level of ventilation of the patient 3 and may be calculated by the control unit 15 in different ways. For example, the minute ventilation may be calculated as the minute ventilation of the patient 3 over a running time window. The time window may have a fixed duration, e.g., 30 seconds, or a variable duration defined by a predetermined number of breaths, e.g., ten breaths.

If the calculated minute ventilation and thus the level of ventilation of the patient 3 falls below a preset threshold value, representing a mandatory minute ventilation (MMV) of the patient 3, the control unit 15 controls the pneumatic unit 17 to deliver a forced breath to the patient 3. Thus, contrary to the unforced bioelectrically triggered breaths, which are delivered to the patient 3 as long as the monitored minute ventilation remains above MMV, the breath that is delivered to the patient 3 when the minute ventilation falls below MMV is a forced breath that is not triggered by the patient.

MMV or any other measure of a minimum mandatory level of ventilation of the patient 3 may be determined by the control unit 15 based on patient data and/or ventilation parameters input by an operator of the breathing apparatus 1, or it may be set manually by the operator.

The minute ventilation of the patient 3 may fall below the desired MMV for several reasons, e.g., if the level of support (e.g., the level of NAVA gain) is set low and the patient's breathing muscles gradually get exhausted. Thus, besides causing delivery of forced breaths when the minute ventilation falls below MMV, the control unit 15 of the breathing apparatus 1 may advantageously be configured to indicate to the operator when the minute ventilation falls below the set MMV, and, optionally, to suggest one or more actions to be taken by the operator to increase the minute ventilation of the patient 3. For example, the control unit 15 may be configured to suggest to the operator, e.g., through the display of a recommendation on a display unit of the breathing apparatus 1, that the support level should be increased. In yet other embodiments, the control unit 15 may be configured to automatically increase the support level if the minute ventilation falls below the set MMV. In an exemplary embodiment of automatic control of the level of support based on the relation between the minute ventilation of the patient 3 and the set MMV, the control unit 15 may be configured to automatically increase the level of support if the minute ventilation repeatedly drops below the set MMV, e.g., for more than a preset number of breaths per minute,

Figs. 2-3 illustrate two exemplary scenarios in which the patient is ventilated using the proposed bioelectrical mode of ventilation with minimum minute ventilation guarantee. The graphs in the drawings represent the inhaled minute ventilation of the patient 3 as a function of time, MV(t), and the line MMV represents the mandatory minute ventilation of the patient.

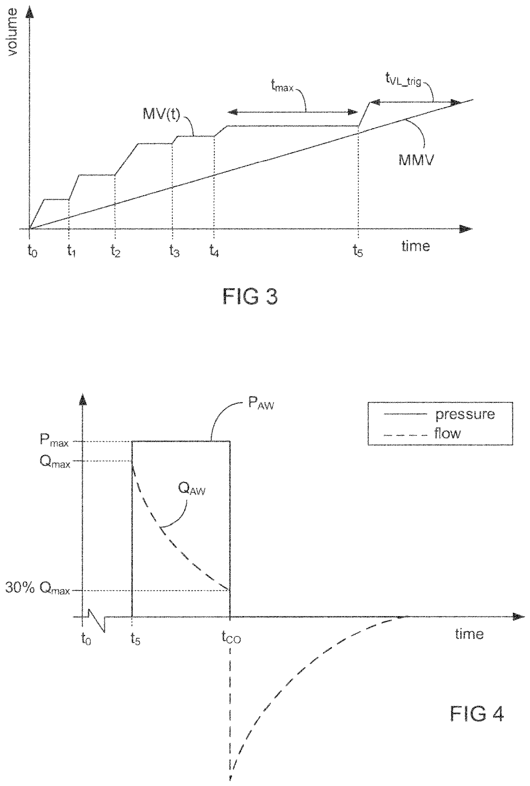

With simultaneous reference to Figs. 2 and 3, bioelectrical trigger events are detected by the control unit 15 at the points in time denoted $t_0$-$t_4$. In response to each bioelectrical trigger event, the control unit 15 controls the pneumatic unit 11 to deliver a breath to the patient 3, causing an increase in the inhaled volume of breathing gas. In this exemplary embodiment, the breaths are delivered in accordance with the well-known principles of NAVA ventilation, meaning that breaths are delivered not only in synchrony with, but also in proportion to the detected bioelectrical signal, e.g., by applying an airway pressure to the patient 3, which is proportional to the magnitude of the bioelectrical signal.

In this exemplary scenario, the unforced breaths that are delivered to the patient 3 in response to bioelectrical trigger events detected at the points in time $t_0$-$t_4$ are sufficient to keep MV(t) above MMV. However, at a point in time $t_5$, MV(t) falls below MMV, which causes delivery of a forced breath to the patient 3.

The forced breath may be any type of breath known in the art of ventilation, such as a pressure-controlled (PCV) breath, a volume-controlled (VCV) breath or a BiPap breath. It may also be a non-patient triggered type of pressure-support breath or volume support breath. The characteristics of the forced breath may, in some embodiments, be independent of the bioelectrical signals captured by the bioelectric sensor 27. In other embodiments, the characteristics of the forced breath may be adapted to the breathing needs of the patient 3 based on bioelectric signals captured prior to delivery of the forced breath by the breathing apparatus 1.

The characteristics of the forced breath may be defined by ventilation parameters including any of, or any combination of, a positive end-expiratory pressure (PEEP), a set inspiratory volume ($V_{insp}$), a set inspiratory pressure ($P_{insp}$), a tidal volume ($V_t$), an inspiratory time ($t_{insp}$) and a duration of an inspiratory pause ($t_{pause}$)

In the illustrated embodiment, the forced breath that is delivered to the patient 3 at the point in time $t_5$ is a forced type of pressure-support breath, the time of delivery of which is determined by the level of ventilation of the patient 3, and the duration of which is determined at least partly by the pulmonary mechanics of the patient. Preferably, the duration of the forced breath is adapted to the pulmonary mechanics of the patient 3 by interrupting the supply of breathing gas when a cycle-off criterion for inspiratory flow is met.

Fig. 4 illustrates a flow-time relationship (dashed line) and a pressure-time relationship (continuous line) for a forced type of pressure-support breath that may be delivered to the patient 3 whenever MV(t) falls below the MMV threshold. When MV(t) reaches the MMV threshold at the point in time $t_5$, the control unit 15 causes breathing gas to be delivered to the patient at an airway pressure $P_{AW}$. The target value for the airway pressure $P_{AW}$ may be predetermined by the control unit based on patient data and/or other ventilation settings, or it may be preset by an operator of the breathing apparatus 1.

In some embodiments, the control unit 15 may be configured to determine a target value for the airway pressure $P_{AW}$ based on the characteristics of a bioelectric signal previously captured by the bioelectric sensor 27. For example, the target value for the airway pressure $P_{AW}$ may be determined by the control unit 15 based on the airway pressure applied to the patient during one or more previous patient-triggered breaths, e.g., the breaths delivered to the patient 3 at the points in time $t_1$-$t_4$, which pressures in turn depend on the bioelectric signal captured at those points in time. With reference to Fig. 1, the airway pressure $P_{AW}$ may be measured by a pressure sensor 37 located in or close to the Y-piece 11 of the patient circuit and coupled to the control unit 15 via a signalling line 39, whereby the control unit 15 may control the supply of breathing gas to the patient 3 based on the pressure measurements obtained by the pressure sensor 37 in order to obtain a desired airway pressure. In addition or in combination to determining the airway pressure $P_{AW}$ of the forced breath based on previously captured bioelectrical signals, the control unit 15 may be configured to determine the tidal volume of the forced breath based on previously captured bioelectrical signals. For example, the control unit 15 may be configured to set the tidal volume of the forced breath 15 to the tidal volume resulting from a previously captured bioelectric signal.

The duration of the forced breath may be determined by a preset cycle-off criterion which, in this exemplary embodiment, is a level of inspiratory flow corresponding to 30% of maximum inspiratory flow, $Q_{max}$. When, at a point in time denoted $t_{co}$ in Fig. 4, the inspiratory flow has decreased to the cycle-off level of 30% of maximum inspiratory flow, the supply of breathing gas to the patient is interrupted and the airway pressure is reduced by means of expiratory gas control means of the breathing apparatus 1 to an expiratory level (which may be zero pressure or a positive end-expiratory pressure), thereby allowing the patient 3 to exhale. As well-known in the art, a cycle-off criterion for inspiratory flow has the effect of adapting the duration of the breath to the pulmonary mechanics of the patient by making the duration of the inspiratory phase dependent on the compliance of the patient's lungs, as well as the actual expiration effort by the patient. The flow used by the control unit 15 to control the duration of forced breaths in accordance with the above described principles may, for example, be an inspiratory flow $Q_{AW}$ measured by the flow sensor 33 in Fig. 1 and substantially corresponding to the flow of gas through the airways of the patient 3.

Consequently, the forced breath that is delivered by the breathing apparatus 1 when the condition for the minimum level of ventilation of the patient 3 is met may be a type of pressure-support breath that is adapted in duration based on the pulmonary mechanics of the patient 3. As mentioned above, the forced breath may also be adapted to the respiratory needs of the patient by adjusting the magnitude of the forced breath (e.g. applied airway pressure) based on at least one bioelectric signal captured during at least one previous patient-triggered breath.

With reference again made to Fig. 3, the proposed bioelectrical mode of ventilation may, additionally, be adapted to provide also for a minimum respiratory rate (RR) guarantee. This may be achieved by employing a trigger condition for time elapsed since delivery of a previous breath, which condition may be used in parallel with the trigger condition for the level of ventilation of the patient 3. In the exemplary scenario illustrated in Fig. 3, a forced breath is delivered at the point in time $t_5$. However, in contrast to the exemplary scenario illustrated in Fig. 2, this is not due to the level of ventilation (MV(t)) of the patient 3 falling below the minimum level of ventilation (MMV). Instead, this is due to the fact that a certain time, $t_{max}$, has elapsed since the time of delivery of the preceding breath, which occurred at the point in time $t_4$. The parameter $t_{max}$, hereinafter referred to as the timeout parameter, sets a maximum time interval between consecutive breaths in the proposed bioelectrically controlled mode of ventilation.

The parameter $t_{max}$ may be automatically determined by the control unit 15 based on patient data and/or ventilation settings, or it may be manually set by an operator of the breathing apparatus 1. In some embodiments, $t_{max}$ may be variable and automatically adjusted by the control unit 15 based on the characteristics of a bioelectric signal previously captured by the bioelectric sensor 27. In this way, the control unit 15 can adjust the mandatory minimum respiratory rate to the expected breathing needs of the patient 3, as reflected by previously detected bioelectrical signals indicative of the patient's breathing efforts.

As clear from the above, the proposed mode of bioelectrically controlled ventilation employs at least two trigger conditions. The first trigger condition is a bioelectrical trigger condition for the bioelectrical signal captured by the bioelectrical sensor 27. The bioelectrical trigger condition may, for example, be a threshold value for the bioelectric signal. When the bioelectrical trigger condition is met, a trigger event of the type "bioelectrical trigger event" has occurred, indicating a desire to breathe by the patient 3. Detection of a bioelectrical trigger event triggers delivery of an unforced, patient-triggered breath, e.g., in the form of a conventional NAVA breath delivered at an airway pressure selected in dependence of the bioelectrical signal that triggers the breath.

The second trigger condition is a trigger condition for the level of ventilation of the patient 3, e.g., a threshold value for the minute ventilation of the patient. When the trigger condition for the level of ventilation of the patient is met, a trigger event of the type "ventilation level trigger event" has occurred, indicating that the level of ventilation of the patient 3 is about to fall below the set minimum ventilation of the patient. Detection of a ventilation level trigger event triggers delivery of a forced, non-patient triggered breath, e.g., a forced pressure-support breath of the type described above.

As discussed above with reference to Fig. 3, the bioelectrically controlled ventilation mode may, in some embodiments, further come with a minimum respiratory rate guarantee. This is achieved by employing a third trigger condition in form of a trigger condition for the time elapsed since delivery of a preceding breath, e.g., by setting a value for the timeout parameter $t_{max}$ defining the maximum time interval between two consecutive breaths. When the trigger condition for the time elapsed since delivery of a preceding breath is met, a trigger event of the type "timeout trigger event" has occurred. Detection of a timeout trigger event triggers delivery of a forced, non-patient triggered breath to the patient, e.g., a forced pressure-support breath of the type described above.

In some embodiments, the bioelectrically controlled ventilation mode may further employ a pneumatic trigger condition, i.e., a trigger condition for a measured pressure and/or flow that changes in response to an effort to breathe by the patient 3 in a manner that renders detection of the breathing effort possible. The measured pressure and/or flow may, for example, be the pressure and/or flow measured by the pressure sensor 37 and/or the flow sensor 33 in Fig. 1. The pneumatic trigger condition may, for example, be a threshold value for the measured pressure or flow. When the pneumatic trigger condition is met, a trigger event of the type "pneumatic trigger event" has occurred. Detection of a pneumatic triggering event may, at least in certain circumstances, trigger delivery of an unforced, patient-triggered breath to the patient.

When employing a pneumatic trigger condition in parallel with the bioelectric trigger condition, the control unit 15 may, in some embodiments, be configured to cause delivery of an unforced, patient-triggered breath on a first-come, first-serve basis in response to detection of any of a bioelectric and pneumatic trigger event.

In other embodiments, the breathing apparatus 1 may be configured to deliver breaths only in response to detection of bioelectrical trigger events (and not pneumatic trigger events) for a first period of time, and to deliver breaths in response to detection of any of a bioelectrical or a pneumatic trigger events during a second period of time following the first period of time.

The pneumatically triggered breaths may be any type of breaths, such as traditional pressure support or volume support breaths.

Determination of when a ventilation level trigger condition or a timeout trigger condition is met can be made in different ways.

In one exemplary embodiment, the control unit is configured to calculate, after each breath delivered by the breathing apparatus (including both unforced patient-triggered breaths and forced non-patient triggered breaths), the time $t_{VL\_trig}$ to the occurrence of a future ventilation level trigger event should the patient not trigger any unforced breath before the time $t_{VL\_trig}$ has lapsed, i.e., the time to delivery of a forced breath due to the level of ventilation of the patient falling below the set minimum level of ventilation. This time, $t_{VL\_trig}$, may be calculated by the control unit 15 from the current level of ventilation, as calculated after delivery of the most recent breath, and the minimum ventilation threshold value.

In an exemplary embodiment, the control unit 15 is configured to calculate $t_{VL\_trig}$ and to start a timer after each breath delivered by the breathing apparatus, and to determine that a ventilation level trigger event has occurred when the timer value has reached $t_{VL\_trig}$. In this way, a forced breath will be delivered to the patient when the current level of ventilation of the patient falls below the set minimum level of ventilation.

To implement the functionality of the respiratory rate guarantee, the control unit 15 may also be configured to determine that a timeout trigger event has occurred when the timer value has reached the set timeout parameter $t_{max}$. In this way a forced breath will be delivered to the patient whenever the timer value reaches any of $t_{VL\_trig}$ and $t_{max}$, whichever occurs first, thus providing for both a minimum minute ventilation guarantee and a minimum respiratory rate guarantee.

According to another embodiment, the control until 15 may be configured to always keep track of the time remaining, $t_{VL\_remain}$, to the occurrence of a future ventilation level trigger event. This may be achieved by the control unit 15 by, at any given point in time, calculate $t_{VL\_remain}$ based on the current level of ventilation of the patient, as calculated by the control unit 15 after delivery of the most recent breath (which may be an unforced patient-triggered breath or a forced non-patient triggered breath), the time elapsed since delivery of the most recent breath, and the minimum ventilation threshold value. If devised as set forth above, the control unit 15 may simply determine $t_{VL\_remain}$ as $t_{VL\_trig}$ minus the current timer value. In this case, to implement the additional minimum respiratory rate guarantee, the control unit 15 may be configured to calculate the time, $t_{remain}$, remaining to delivery of a future forced breath, no matter whether the breath is triggered by the occurrence of a ventilation level trigger event or a timeout trigger event, as: $t_{remain} = t_{VL\_trig}$- timer value, if

$$t_{VL\_trig} < t_{max}, \text{ and} \qquad \text{(Eq. 1)}$$

$$t_{remain} = t_{max} - \text{timer value, if } t_{VL\_trig} > t_{max}, \qquad \text{(Eq. 2)}$$

and to deliver a forced breath to the patient when $t_{remain}$ is zero.

It should be appreciated that any unforced, patient-triggered breath causes an increase in the level of ventilation of the patient 3, and so has the effect of prolonging $t_{remain}$ and thus postponing the delivery of forced, non-patient triggered breaths to the patient 3. By configuring the control unit 15 as set forth above, spontaneous breathing of the patient 3 is encouraged and forced breaths are delivered only if required to maintain the level of ventilation of the patient 3 above the set minimum level of ventilation, or to avoid too long time intervals between consecutive breaths.

In some embodiments, the control unit 15 may further be configured to ignore detection of bioelectric trigger events occurring at points in time where the level of ventilation of the patient is close to the minimum ventilation threshold value, e.g., when MV(t) is close to MMV. For example, the control unit may be configured to ignore bioelectric trigger events when the time remaining until delivery of a forced breath, $t_{remain}$, has fallen below a predetermined threshold value, e.g. a threshold value of 1 second. This has the effect of avoiding situations in which a small unforced breath (e.g. caused by a weak bioelectrical trigger event) is immediately followed by a bigger forced breath (e.g. caused by a ventilation level trigger event), which situations may cause discomfort to the patient and render difficult proper flow and pressure regulation in the breathing apparatus 1.

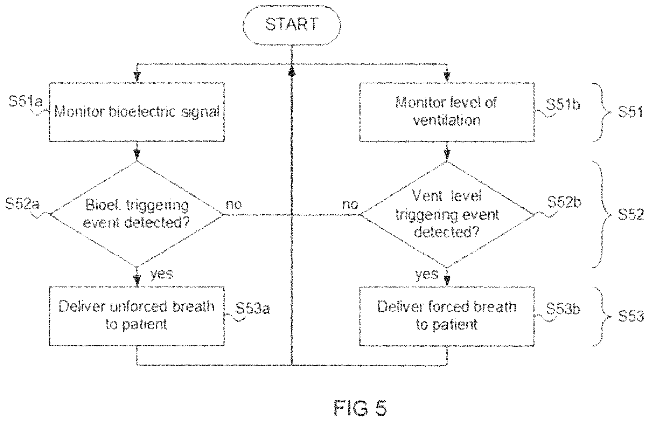

Fig. 5 is a flow chart illustrating a method of providing bioelectrically controlled ventilation to a patient according to an exemplary embodiment of the present disclosure.

The patient is ventilated in a ventilation mode wherein an unforced patient-triggered breath is delivered to the patient upon detection of a bioelectrical trigger event indicative of a spontaneous breathing effort by the patient, and wherein a forced non-patient triggered breath is delivered to the patient upon detection of a ventilation level trigger event indicating that the level of ventilation of the patient, e.g., the minute ventilation (MV) of the patient, has fallen below a minimum ventilation threshold value, e.g., a set minimum minute ventilation (MMV) of the patient.

In a first step, S51, a bioelectrical signal indicative of spontaneous breathing efforts by the patient is monitored (S51a) together with a quantity indicative of the level of ventilation of the patient (S51b), such as a calculated minute ventilation of the patient.

In a second step, S52, it is determined whether a bioelectric trigger event has occurred (S52a), or if a ventilation level trigger event has occurred (S52b). As discussed above, a bioelectric trigger event may be detected, e.g., by determining whether the amplitude of the bioelectric signal exceeds a certain threshold value (trigger threshold), and a ventilation level trigger event may be detected, e.g., by determining whether the minute ventilation of the patient has fallen below a set minimum minute ventilation.

If, in step S52a, it is determined that a bioelectric trigger event has occurred, the method proceeds to step S53a in which an unforced and patient-triggered breath is delivered to the patient. As discussed above, this breath may for example be the type of breath normally delivered to patients ventilated in NAVA mode, i.e., a breath that is delivered in synchrony with and in proportion to the bioelectric signal.

If, on the other hand, a ventilation level trigger event is detected in step S52b, the method proceeds to step S53b in which a forced and non-patient triggered breath is delivered to the patient. As discussed above, this breath may for example be a forced type of pressure-support breath with a cycle-off criterion for inspiratory flow.

The method then starts over again by monitoring the bioelectric signal and the level of ventilation of the patient.

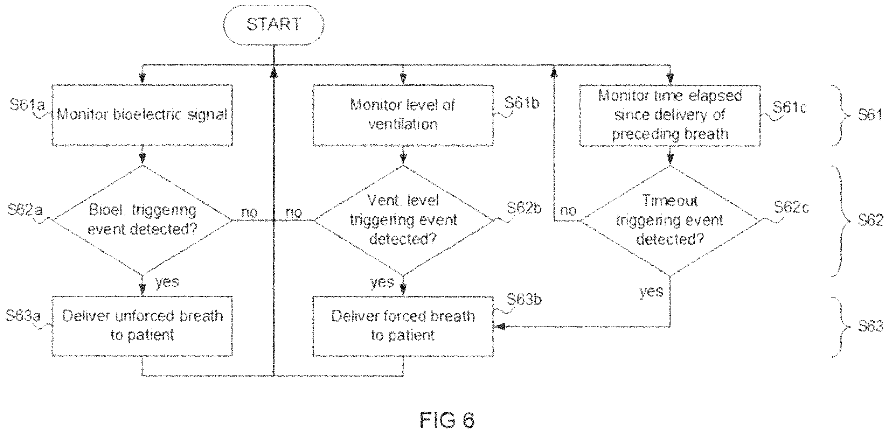

Fig. 6 is a flow chart illustrating a method of providing bioelectrically controlled ventilation to a patient according to another exemplary embodiment of the present disclosure.

The method illustrated in Fig. 6 differs from the method illustrated in Fig. 5 only by employing an additional trigger condition for delivery of forced, non-patient triggered breaths. This trigger condition is the above discussed trigger condition for the time elapsed since delivery of a preceding breath, which trigger condition serves to set a maximum time interval between consecutive breaths.

In a first step, S61, the time elapsed since delivery of the preceding breath is monitored (S61c) in parallel with the bioelectric signal (step S61a, corresponding to step S51a) and the level of ventilation of the patient (step S62b, corresponding to step S51b).

In a second step, S62, it is determined if a bioelectric trigger event has occurred (step S62a, corresponding to step S52a), if a ventilation level trigger event has occurred (step S62b, corresponding to step S52b), or if a timeout trigger event has occurred (S62c). As discussed above, a timeout trigger event has occurred when the time elapsed since delivery of a preceding breath has reached a set maximum value, defined by the timeout parameter $t_{max}$.

If, in step S62a, it is determined that a bioelectric trigger event has occurred, the method proceeds to step S63a, corresponding to step S53a in Fig. 5.

If, in step S62b, it is determined that a ventilation level trigger event has occurred, the method proceeds to step S63b, corresponding to step S53b in Fig. 5.

If, in step S62c, it is determined that a timeout trigger event has occurred, the method also proceeds to step S63b, meaning that a forced, non-patient triggered breath, e.g., a forced type of pressure-support breath with a cycle-off criterion for inspiratory flow, is delivered to the patient.

The method then starts over again by monitoring the bioelectric signal, the level of ventilation of the patient, and the time elapsed since delivery of the most recently delivered breath.

**Claims**

1. A breathing apparatus (1) configured to provide bioelectrically controlled ventilation to a patient (3) by ventilating the patient in a ventilation mode in which patient-triggered breaths are delivered to the patient upon detection of bioelectrical trigger events indicative of spontaneous breathing efforts by the patient, the breathing apparatus (1) comprising a bioelectric sensor arrangement (27) configured to detect a bioelectric signal indicative of the patient's effort to breathe, and a control unit (15) configured to control the ventilation of the patient (3) based on the bioelectric signal detected by the bioelectric sensor arrangement, wherein a bioelectrical trigger event occurs when the bioelectric signal meets a bioelectrical trigger condition, **characterised in that** the breathing apparatus (1) is configured to monitor a level of ventilation of the patient, and to deliver a non-patient triggered breath to the patient when the level of ventilation falls below a minimum ventilation threshold value, wherein the non-patient triggered breath is independent of the bioelectric signal and is forced onto the patient (3) when the monitored level of ventilation falls below the minimum ventilation threshold value.

2. The breathing apparatus (1) of claim 1, wherein the breathing apparatus is configured to use a measure of the minute ventilation (MV) of the patient (3) as indicator of the level of ventilation of the patient, and to deliver a non-patient triggered breath to the patient when the minute ventilation falls below a minimum minute ventilation threshold value (MMV).

3. The breathing apparatus (1) of any of the preceding claims, wherein the breathing apparatus is configured to deliver a non-patient triggered breath to the patient when a certain period of time ($t_{max}$) has elapsed since a preceding breath was delivered to the patient.

4. The breathing apparatus (1) of any of the preceding claims, wherein the non-patient triggered breath is a breath having a duration that is adapted to the pulmonary mechanics of the patient (3).

5. The breathing apparatus (1) of claim 4, wherein the breathing apparatus is configured to adapt the duration of the non-patient triggered breath to the pulmonary mechanics of the patient by employing a cycle-off criterion for inspiratory flow.

6. A computer program for causing a breathing apparatus (1) to provide bioelectrically controlled ventilation to a patient (3) by ventilating the patient in a ventilation mode in which patient-triggered breaths are delivered (S53a; S63a) to the patient upon detection (S52a; S62a) of bioelectrical trigger events indicative of spontaneous breathing efforts by the patient, wherein a bioelectric trigger event occurs when a bioelectric signal indicative of the patient's effort to breathe, detected by a bioelectric sensor arrangement (27) of the breathing apparatus (1), meets a bioelectric trigger condition, **characterised in that** the computer program comprises computer-readable code segments which, when executed by a processor (23) of the breathing apparatus, causes the breathing apparatus to:

- monitor (S51b; S61b) a level of ventilation of the patient, and
- deliver (S53b; S63b) a non-patient triggered breath to the patient when the level of ventilation falls below a minimum ventilation threshold value, wherein the non-patient triggered breath is independent of the bioelectric signal and is forced onto the patient (3) when the monitored level of ventilation falls below the minimum ventilation threshold value.

7. The computer program of claim 6, wherein the computer-readable code segments, when executed by the processor (23), cause the breathing apparatus (1) to:

- monitor a measure of the minute ventilation (MV) of the patient (3) as indicator of the level of ventilation, and
- deliver the non-patient triggered breath to the patient when the minute ventilation falls below a minimum minute ventilation threshold value (MMV).

8. The computer program of claim 6 or 7, wherein the computer-readable code segments, when executed by the processor (23), cause the breathing apparatus (1) to:

- deliver a non-patient triggered breath when a certain period of time ($t_{max}$) has elapsed since a preceding breath was delivered to the patient.

9. The computer program of any of the claims 6 to 8, wherein the non-patient triggered breath is a breath having a duration that is adapted to the pulmonary mechanics of the patient (3).

10. The computer program of claim 9, wherein the computer-readable code segments, when executed by the processor (23), cause the breathing apparatus (1) to adapt the duration of the non-patient triggered breath to the pulmonary mechanics of the patient by employing a cycle-off criterion for inspiratory flow.

**Patentansprüche**

1. Beatmungsgerät (1), das ausgelegt ist, um einem Patienten (3) bioelektrisch gesteuerte Beatmung dadurch bereitzustellen, dass der Patient in einer Atembetriebsart beatmet wird, in welcher bei der Detektion von bioelektrischen Trigger-Ereignissen, die spontane Atemanstrengungen des Patienten anzeigen, Patient-getriggerte Atemzüge an den Patienten geliefert werden, wobei das Beatmungsgerät (1) eine bioelektrische Sensoranordnung (27) umfasst, die ausgelegt ist, ein bioelektrisches Signal, das die Atemanstrengung des Patienten anzeigt, zu detektieren, und eine Steuereinheit (15) umfasst, die zum Steuern der Beatmung des Patienten (3) basierend auf dem durch die bioelektrische Sensoranordnung detektierten bioelektrischen Signal ausgelegt ist, wobei ein bioelektrisches Trigger-Ereignis auftritt, wenn das bioelektrische Signal eine bioelektrische Trigger-Voraussetzung erfüllt, **dadurch gekennzeichnet, dass** das Beatmungsgerät (1) dazu ausgelegt ist, den Beatmungsgrad des Patienten zu überwachen und einen nicht-Patient-getriggerten Atemzug an den Patienten bereitzustellen, wenn der Beatmungsgrad unter einen minimalen Beatmungsschwellenwert sinkt, wobei der nicht-Patient-getriggerte Atemzug von dem bioelektrischen Signal unabhängig ist und auf den Patienten (3) gezwungen wird, wenn der überwachte Beatmungsgrad unter den minimalen Beatmungsschwellenwert sinkt.

2. Beatmungsgerät (1) nach Anspruch 1, wobei das Beatmungsgerät dazu ausgelegt ist, eine Messung des Atemminutenvolumens (MV) des Patienten (3) als Indikator für den Beatmungsgrad des Patienten zu verwenden und einen nicht-Patient-getriggerten Atemzug an den Patienten bereitzustellen, wenn das Atemminutenvolumen unter einen minimalen Atemminutenvolumenschwellenwert (MMV) sinkt.

3. Beatmungsgerät (1) nach einem der vorgehenden Ansprüche, wobei das Beatmungsgerät ausgelegt ist, um einen nicht-Patient-getriggerten Atemzug an den Patienten bereitzustellen, wenn eine bestimmte Zeitdauer ($t_{max}$) verstri-

chen ist, seit ein vorausgehender Atemzug an den Patienten geliefert wurde.

4. Beatmungsgerät (1) nach einem der vorgehenden Ansprüche, wobei der nicht-Patient-getriggerte Atemzug ein Atemzug ist, der eine Dauer aufweist, die an die Lungenmechanismen des Patienten (3) angepasst ist.

5. Beatmungsgerät (1) nach Anspruch 4, wobei das Beatmungsgerät ausgelegt ist, um die Dauer des nicht-Patient-getriggerten Atemzugs an die Lungenmechanismen des Patienten durch Verwendung eines Cycle-Off-Kriteriums für den inspiratorischen Flow anzupassen.

6. Computerprogramm für das Veranlassen eines Beatmungsgeräts (1) dazu, bioelektrisch gesteuerte Beatmung an einen Patienten (3) dadurch bereitzustellen, dass der Patient in einer Beatmungsbetriebsart beatmet wird, in welcher Patient-getriggerte Atemzüge bei der Detektierung (S52a; S62a) von bioelektrischen Trigger-Ereignissen, die spontane Atemanstrengungen durch den Patienten anzeigen, an den Patienten (S53a; S63a) geliefert werden, wobei ein bioelektrisches Trigger-Ereignis auftritt, wenn ein bioelektrisches Signal, das die Atemanstrengung des Patienten anzeigt, die durch eine bioelektrische Sensoranordnung (27) des Beatmungsgeräts (1) detektiert wird, eine bioelektrische Trigger-Voraussetzung erfüllt, **dadurch gekennzeichnet, dass** das Computerprogramm computerlesbare Codesegmente umfasst, die bei Ausführung durch einen Prozessor (23) des Beatmungsgeräts das Beatmungsgerät veranlassen zum:

   - Überwachen (S51b; S61b) eines Beatmungsgrades des Patienten, und
   - Bereitstellen (S53b; S63b) eines nicht-Patient-getriggerten Atemzugs an den Patienten, wenn der Beatmungsgrad unter einen minimalen Beatmungsschwellenwert sinkt, wobei der nicht-Patient-getriggerte Atemzug von dem bioelektrischen Signal unabhängig ist und auf den Patienten (3) gezwungen wird, wenn der überwachte Beatmungsgrad unter den minimalen Beatmungsschwellenwert sinkt.

7. Computerprogramm nach Anspruch 6, wobei die computerlesbaren Codesegmente bei Ausführung durch den Prozessor (23) das Beatmungsgerät (1) veranlassen zum:

   - Überwachen einer Messung des Atemminutenvolumens (MV) des Patienten (3) als Indikator für den Atembeatmungsgrad, und
   - Bereitstellen des nicht-Patient-getriggerten Atemzugs an den Patienten, wenn das Atemminutenvolumen unter einen minimalen Atemminutenvolumenschwellenwert (MMV) sinkt.

8. Computerprogramm nach Anspruch 6 oder 7, wobei die computerlesbaren Codesegmente bei Ausführung durch den Prozessor (23) das Beatmungsgerät (1) veranlassen zum:

   - Bereitstellen eines nicht-Patient-getriggerten Atemzugs, wenn eine bestimmte Zeitdauer ($t_{max}$) verstrichen ist, seit ein vorausgehender Atemzug an den Patienten geliefert wurde.

9. Computerprogramm nach Anspruch 6 bis 8, wobei der nicht-Patient-getriggerte Atemzug ein Atemzug ist, der eine Dauer aufweist, die an die Lungenmechanismen des Patienten (3) angepasst ist.

10. Computerprogramm nach Anspruch 9, wobei die computerlesbaren Codesegmente bei Ausführung durch den Prozessor (23) das Beatmungsgerät (1) dazu veranlassen, die Dauer des nicht-Patient-getriggerten Atemzugs an die Lungenmechanismen des Patienten durch Verwendung eines Cycle-Off-Kriteriums für den inspiratorischen Flow anzupassen.

**Revendications**

1. Appareil respiratoire (1) configuré pour fournir une ventilation à commande bioélectrique à un patient (3) en ventilant le patient dans un mode de ventilation dans lequel des respirations déclenchées par le patient sont délivrées au patient lors de la détection d'événements déclencheurs bioélectriques indicatifs d'efforts respiratoires spontanés par le patient, l'appareil respiratoire (1) comprenant un agencement de capteur bioélectrique (27) configuré pour détecter un signal bioélectrique indicatif de l'effort du patient à respirer, et une unité de commande (15) configurée pour commander la ventilation du patient (3) sur la base du signal bioélectrique détecté par l'agencement de capteur bioélectrique, un événement déclencheur bioélectrique se produisant lorsque le signal bioélectrique satisfait une condition de déclenchement bioélectrique, **caractérisé en ce que** l'appareil respiratoire (1) est configuré pour surveiller un niveau de ventilation du patient, et pour délivrer au patient une respiration non déclenchée par le patient lorsque le niveau de ventilation tombe en dessous d'une valeur seuil de ventilation minimale, la respiration non déclenchée par le patient étant indépendante du signal bioélectrique et étant forcée sur le patient (3) lorsque le niveau de ventilation surveillé tombe au-dessous de la valeur seuil de ventilation minimale.

**2.** Appareil respiratoire (1) selon la revendication 1, dans lequel l'appareil respiratoire est configuré pour utiliser une mesure de la ventilation minute (MV) du patient (3) en tant qu'indicateur du niveau de ventilation du patient, et pour délivrer au patient une respiration non déclenchée par le patient lorsque la ventilation minute tombe en dessous d'une valeur seuil de ventilation minute minimale (MMV).

**3.** Appareil respiratoire (1) selon l'une quelconque des revendications précédentes, dans lequel l'appareil respiratoire est configuré pour fournir au patient une respiration non déclenchée par le patient lorsqu'une certaine période de temps ($t_{max}$) s'est écoulée depuis qu'une respiration précédente a été délivrée au patient.

**4.** Appareil respiratoire (1) selon l'une quelconque des revendications précédentes, dans lequel la respiration non déclenchée par le patient est une respiration ayant une durée qui est adaptée à la mécanique pulmonaire du patient (3).

**5.** Appareil respiratoire (1) selon la revendication 4, dans lequel l'appareil respiratoire est configuré pour adapter la durée de la respiration non déclenchée par le patient à la mécanique pulmonaire du patient en employant un critère d'arrêt de cycle pour le débit inspiratoire.

**6.** Programme informatique destiné à amener un appareil respiratoire (1) à fournir une ventilation à commande bioélectrique à un patient (3) en ventilant le patient dans un mode de ventilation dans lequel des respirations déclenchées par le patient sont délivrées (S53a; S63a) au patient lors de la détection (S52a; S62a) d'événements déclencheurs bioélectriques indicatifs d'efforts respiratoires spontanés par le patient,

un événement déclencheur bioélectrique se produisant lorsqu'un signal bioélectrique indicatif de l'effort du patient à respirer, détecté par un agencement de capteur bioélectrique (27) de l'appareil respiratoire (1), satisfait une condition de déclenchement bioélectrique,
**caractérisé en ce que** le programme informatique comprend des segments de code lisibles par ordinateur qui, lorsqu'ils sont exécutés par un processeur (23) de l'appareil respiratoire, amènent l'appareil respiratoire à exécuter les étapes consistant à :

- surveiller (S51b; S61b) un niveau de ventilation du patient, et
- délivrer (S53b; S63b) au patient une respiration non déclenchée par le patient lorsque le niveau de ventilation tombe en dessous d'une valeur seuil de ventilation minimale, la respiration non déclenchée par le patient étant indépendante du signal bioélectrique et étant forcée sur le patient (3) lorsque le niveau de ventilation surveillé tombe au-dessous de la valeur seuil de ventilation minimale.

**7.** Programme informatique selon la revendication 6, dans lequel les segments de code lisibles par ordinateur, lorsqu'ils sont exécutés par le processeur (23), amènent l'appareil respiratoire (1) à :

- surveiller une mesure de la ventilation minute (MV) du patient (3) en tant qu'indicateur du niveau de ventilation, et
- délivrer au patient la respiration non déclenchée par le patient lorsque la ventilation minute tombe en dessous d'une valeur seuil de ventilation minute minimale (MMV).

**8.** Programme informatique selon la revendication 6 ou 7, dans lequel les segments de code lisibles par ordinateur, lorsqu'ils sont exécutés par le processeur (23), amènent l'appareil respiratoire (1) à :

- délivrer une respiration non déclenchée par le patient lorsqu'une certaine période de temps ($t_{max}$) s'est écoulée depuis qu'une respiration précédente a été délivrée au patient.

**9.** Programme informatique selon l'une quelconque des revendications 6 à 8, dans lequel la respiration non déclenchée par le patient est une respiration ayant une durée qui est adaptée à la mécanique pulmonaire du patient (3).

**10.** Programme informatique selon la revendication 9, dans lequel les segments de code lisibles par ordinateur, lorsqu'ils sont exécutés par le processeur (23), amènent l'appareil respiratoire (1) à adapter la durée de la respiration non déclenchée par le patient à la mécanique pulmonaire du patient en employant un critère d'arrêt de cycle pour le débit inspiratoire.

FIG 1

FIG 2

FIG 3

FIG 4

FIG 5

FIG 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 199848877 A
- WO 199962580 A
- WO 2006131149 A
- WO 2008131798 A
- US 8469026 B
- SE 2015050369 W

**Non-patent literature cited in the description**

- **GRAVE DE PERALTA et al.** Patient Machine Interface for the Control of Mechanical Ventilation Devices. *Brain Sci.,* 2013, vol. 3, 1554-1568